# EUROPÄISCHE PATENTSCHRIFT

(11) **EP 0 257 048 B1**
(45) Veröffentlichungstag und Bekanntmachung des Hinweises auf die Patenterteilung: **24.08.1994**
(21) Anmeldenummer: 87901018.9
(22) Anmeldetag: 31.01.1987
(51) Int. Cl.: C07D 239/26, C07D 241/12, C07C 255/03, C07C 255/46, C07C 255/11, C07C 255/15, C07D 213/30, C07C 69/24, C07D 213/55, C07C 69/92

(54) **OPTISCH AKTIVE VERBINDUNGEN**
OPTICALLY ACTIVE COMPOUNDS
COMPOSES OPTIQUEMENT ACTIFS

(30) Priorität: 17.02.1986 DE 3604905; 10.09.1986 DE 3630771
(43) Veröffentlichungstag der Anmeldung: 02.03.1988
(73) Patentinhaber: MERCK PATENT GmbH, D-64271 Darmstadt (DE)
(72) Erfinder: EIDENSCHINK, Rudolf, D-6109 Mühltal (DE); ESCHER, Claus, D-6109 Mühltal (DE); GEELHAAR, Thomas, D-6500 Mainz (DE); HITTICH, Reinhard, D-6101 Modautal 1 (DE); KURMEIER, Hans-Adolf, D-6104 Seeheim-Jugenheim (DE); PAULUTH, Detlef, D-6100 Darmstadt (DE); WÄCHTLER, Andreas, D-6103 Griesheim (DE)
(86) Internationale Anmeldenummer: DE8700036
(87) Internationale Veröffentlichungsnummer: WO8705018

(56) Entgegenhaltungen:
- EP-A- 014 885
- EP-A- 138 006
- EP-A- 168 043
- EP-A- 0 110 299
- EP-A- 0 136 725
- EP-A- 0 163 229
- EP-A- 0 194 659
- EP-A- 0 213 841
- WO-A-86/00087
- WO-A-86/04328
- WO-A-86/06373
- WO-A-87/01717
- Chem. Abstr. Vol. 102, Nr. 95 409b
- Chem. Abstr. Vol. 101, Nr. 6 821u

## Beschreibung

Die Erfindung betrifft optisch aktive Verbindungen der Formel I

R¹-Q¹-C*HX-Q²-A⁴-Z¹-A²-(Z²-A³)ₙ-R² I

worin
- R²: eine geradkettige oder verzweigte Alkyl- oder Perfluoralkyl-Gruppe mit jeweils 1-12 C-Atomen, worin auch eine oder mehrere CH₂- bzw. CF₂-Gruppen durch eine Gruppierung ausgewählt aus der Gruppe -O-, -S-, -CO-, -O-CO-, -S-CO-, -O-COO-, -CO-O-, -CO-S-, -CH=CH-, -CHHalogen- und -CHCN- oder auch durch eine Kombination von zwei geeigneten Gruppierungen ersetzt sein können, wobei zwei Heteroatome nicht direkt miteinander verknüpft sind, F, Cl, Br, CN oder auch H,
- A², A³ und A⁴: jeweils unsubstituiertes oder durch ein oder zwei F- und/oder Cl-Atome und/oder CH₃-Gruppen und/oder CN-Gruppen substituiertes 1,4-Phenylen, worin auch eine oder zwei CH-Gruppen durch N ersetzt sein können, 1,4-Cyclohexylen, worin auch ein oder zwei nicht benachbarte CH₂-Gruppen durch O-Atome und/oder S-Atome ersetzt sein können, Piperidin-1,4-diyl, 1,4-Bicyclo(2,2,2)-octylen-, Naphthalin-2,6-diyl-, Decahydronaphthalin-2,6-diyl- oder 1,2,3,4-Tetrahydronaphthalin-2,6-diyl-Gruppen,
- Z¹ und Z²: jeweils -CO-O-, -CO-S-, -O-CO-, -S-CO-, -CH₂CH₂-, -OCH₂-, -CH₂O-, -C≡C- oder eine Einfachbindung,
- X: Halogen oder CN
- n: 0 oder 1,
- Q²: -(CH₂)ₚ-, -(CH₂)ₚ-CO-O-, -(CH₂)ₚ-O-CO- oder -(CH₂)ₚ-O-, wobei p 1 bis 3 bedeutet, oder eine Einfachbindung
und
R¹-Q¹- 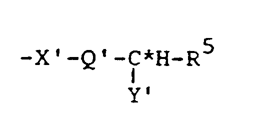 bedeutet, worin
- X': -CO-O-, -O-CO-, -O-CO-O-, -CO-, -O-, -S-, -CH=CH-, -CH=CH-COO- oder eine Einfachbindung,
- Q': Alkylen mit 1 bis 5 C-Atomen, worin auch eine nicht mit X' verknüpfte CH₂-Gruppe durch -O-, -CO-, -O-CO-, -CO-O- oder -CH=CH- ersetzt sein kann, oder eine Einfachbindung,
- Y': CN oder Halogen und
- R⁵: eine Alkylgruppe mit 1 bis 15 C-Atomen, worin auch eine oder zwei nicht benachbarte CH₂-Gruppen durch -O-, -CO-, -O-CO-, -CO-O- und/oder -CH=CH- ersetzt sein können,
bedeutet.

Die Verbindungen der Formel I können wie ähnliche in DE-OS 35 15 373 beschriebene Verbindungen als Komponenten chiraler getilteter smektischer flüssigkristalliner Phasen verwendet werden. Weitere ähnliche Verbindungen sind aus EP-A-138 006 bekannt.
Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44, (lett.), L-771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiralen getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 »m) unterdrückt. Dadurch werden die Längsachsen der Moleküle gezwungen, sich in einer Ebene parallel zu den Platten der Zelle anzuordnen, wodurch Zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. Sc*) ist deren relativ hohe optische Anisotropie, die durch relativ hohe Viskositätswerte und/oder relativ niedrige Werte für die Spontanpolarisation bedingten nicht ausreichend kurzen Schaltzeiten, sowie, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/Florida, April/Mai 1985, Vortrag 8.3).

Es wurde nun gefunden, daß die Verwendung von Verbindungen der Formel I als Komponenten chiraler getilteter smektischer Mischungen die erwähnten Nachteile wesentlich vermindern kann. Die Verbindungen der Formel I sind somit als Komponenten chiraler getilteter smektischer flüssigkristalliner Phasen vorzüglich geeignet. Insbesondere sind mit ihrer Hilfe chemisch besonders stabile chirale getiltete smektische flüssigkristalline Phasen mit günstigen ferroelektrischen Phasenbereichen, insbesondere mit breiten Sc*-Phasenbereichen, sehr günstigem Verhältnis der Rotationsviskosität zur Spontanpolarisation (γ/P), negativer oder auch positiver dielektrischer Anisotropie, niedriger optischer Anisotropie, günstiger Pitchhöhe und für derartige Phasen hohen Werten für die spontane Polarisation und sehr kurzen Schaltzeiten herstellbar. P ist die spontane Polarisation in nC/cm².

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch und/oder die Schaltzeiten einer solchen Phase zu variieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos, weisen relativ niedrige Werte der optischen Anisotropie und sehr günstige Werte für γ/P auf. Teilweise zeigen die Verbindungen der Formel I flüssigkristalline Mesophasen in einem für die elektrooptische Verwendung günstig gelegenen Temperaturbereich, es können jedoch auch isotrope oder monotrop flüssigkristalline Verbindungen der Formel I als Komponenten chiraler getilteter smektischer Phasen vorteilhaft eingesetzt werden. Chemisch, thermisch und gegen Licht sind sie sehr stabil.

Gegenstand der Erfindung sind somit die optisch aktiven Verbindungen der Formel I, die Verwendung der Verbindungen der Formel I als Komponenten flüssigkristalliner Phasen, sowie chirale getiltete smektische flüssigkristalline Phasen mit einem Gehalt an mindestens einer optisch aktiven Verbindung der Formel I.

Gegenstand der Erfindung sind ferner elektrooptische Anzeigeelemente, die derartige Phasen enthalten.

Der Einfachheit halber bedeuten im folgenden Ph eine 1,4-Phenylengruppe, worin auch eine oder zwei CH-Gruppen durch N ersetzt sein können, Cy eine 1,4-Cyclohexylengruppe, worin auch eine oder zwei nicht benachbarte CH₂-Gruppen durch O-Atome ersetzt sein können und Bi eine Bicyclo(2,2,2)octylengruppe.

Vor- und nachstehend haben R¹, R², n, A², A³, A⁴, Q¹, Q², X, Z¹ und Z² die angegebene Bedeutung, sofern nicht ausdrücklich etwas anderes vermerkt ist. C* ist ein asymmetrisches Kohlenstoffatom.

Die Verbindungen der Formel I umfassen dementsprechend insbesondere Verbindungen der Teilformeln Ia und Ib (mit zwei Ringen)

R¹-Q¹-C*HX-Q²-A⁴-A²-R² Ia

R¹-Q¹-C*HX-Q²-A⁴-Z¹-A²-R² Ib

und Ic bis If (mit drei Ringen):

R¹-Q¹-C*HX-Q²-A⁴-A²-A³-R² Ic

R¹-Q¹-C*HX-Q²-A⁴-A²-Z²-A³-R² Id

R¹-Q¹-C*HX-Q²-A⁴-Z¹-A²-A³-R² Ie

R¹-Q¹-C*HX-Q²-A⁴-Z¹-A²-Z²-A³-R² If

Darunter sind diejenigen der Formeln Ia, Ib, Ic, Id und Ie besonders bevorzugt.

Die bevorzugten Verbindungen der Formel Ia umfassen solche der Teilformeln Ia1 bis Ia4:

R¹-Q¹-C*HX-Q²-Ph-Ph-R² Ia1

R¹-Q¹-C*HX-Q²-Cy-Ph-R² Ia2

R¹-Q¹-C*HX-Q²-Ph-Cy-R² Ia3

R¹-Q¹-C*HX-Q²-Cy-Cy-R² Ia4

Darunter sind diejenigen der Formeln Ia1 und Ia3 besonders bevorzugt.

Die bevorzugten Verbindungen der Formel Ib umfassen solche der Teilformeln Ib1 bis Ib4:

R¹-Q¹-C*HX-Q²-Ph-Z¹-Ph-R² Ib1

R¹-Q¹-C*HX-Q²-Cy-Z¹-Ph-R² Ib2

R¹-Q¹-C*HX-Q²-Ph-Z¹-Cy-R² Ib3

R¹-Q¹-C*HX-Q²-Cy-Z¹-Cy-R² Ib4

Darunter sind diejenigen der Formeln Ib1 und Ib3 besonders bevorzugt.

Die bevorzugten Verbindungen der Formel Ic umfassen solche der Teilformeln Ic1 bis Ic4:

R¹-Q¹-C*HX-Q²-Ph-Ph-Cy-R² Ic1

R¹-Q¹-C*HX-Q²-Ph-Cy-Cy-R² Ic2

R¹-Q¹-C*HX-Q²-Ph-Cy-Ph-R² Ic3

R¹-Q¹-C*HX-Q²-Ph-Ph-Ph-R² Ic4

Die bevorzugten Verbindungen der Formel Id umfassen solche der Teilformeln Id1 bis Id3:

R¹-Q¹-C*HX-Q²-Ph-Ph-Z²-Cy-R² Id1

R¹-Q¹-C*HX-Q²-Ph-Cy-Z²-Ph-R² Id2

R¹-Q¹-C*HX-Q²-Ph-Ph-Z²-Ph-R² Id3

Die bevorzugten Verbindungen der Formel Ie umfassen solche der Teilformeln Ie1 bis Ie5:

R¹-Q¹-C*HX-Q²-Ph-Z¹-Ph-Ph-R² Ie1

R¹-Q¹-C*HX-Q²-Ph-Z¹-Ph-Cy-R² Ie2

R¹-Q¹-C*HX-Q²-Ph-Z¹-Cy-Ph-R² Ie3

R¹-Q¹-C*HX-Q²-Ph-Z¹-Cy-Cy-R² Ie4

R¹-Q¹-C*HX-Q²-Cy-Z¹-Ph-Ph-R² Ie5

Bevorzugt sind Verbindungen der vor- und nachstehenden Formeln worin R² Alkyl, -O-Alkyl, -C0-Alkyl, -C0-0-Alkyl, -OCO-Alkyl oder -OCOO-Alkyl bedeutet.

In den bevorzugten Verbindungen der vor- und nachstehenden Formeln können die Alkylreste, in denen auch eine CH₂-Gruppe (Alkoxy bzw. Oxaalkyl) durch ein 0-Atom ersetzt sein kann, geradkettig oder verzweigt sein. Vorzugsweise haben sie 5, 6, 7, 8, 9 oder 10 C-Atome und bedeuten demnach bevorzugt Pentyl, Hexyl, Heptyl, Octyl, Nonyl, Decyl, Pentoxy, Hexoxy, Heptoxy, Octoxy, Nonoxy oder Decoxy, ferner auch Ethyl, Propyl, Butyl, Undecyl, Dodecyl, Propoxy, Ethoxy, Butoxy, Undecoxy, Dodecoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6- Oxaheptyl.

A², A³ und A⁴ sind bevorzugt Cy oder Ph. In den Verbindungen der vor- und nachstehenden Formeln bedeutet Ph vorzugsweise eine 1,4-Phenylen- (Phe), eine Pyrimidin-2,5-diyl- (Pyr), eine Pyridin-2,5-diyl- (Pyn), eine Pyrazin-3,6-diyl- oder eine Pyridazin-2,5-diyl-Gruppe, insbesondere bevorzugt Phe, Pyr oder Pyn. Vorzugsweise enthalten die erfindungsgemäßen Verbindungen nicht mehr als eine 1,4-Phenylengruppe, worin eine oder zwei CH-Gruppen durch N ersetzt sind. Cy bedeutet vorzugsweise eine trans-1,4-Cyclohexylengruppe. Insbesondere bevorzugt sind jedoch Verbindungen der Formel I, worin eine der Gruppen A², A³ und A⁴ eine in 1- oder 4-Position durch CN substituierte 1,4-Cyclohexylengruppe bedeutet und die Nitrilgruppe sich in axialer Position befindet, d.h. die Gruppe A², A³ bzw. A⁴ die folgende Konfiguration aufweist: 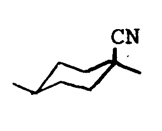
Besonders bevorzugt sind Verbindungen der Formel I und der vorstehenden Teilformeln, die eine Gruppierung -Ph-Ph- enthalten. -Ph-Ph- ist vorzugsweise -Phe-Phe-, Phe-Pyr oder Phe-Pyn. Besonders bevorzugt sind die Gruppen 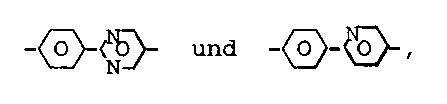
insbesondere 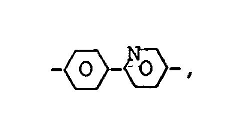
sowie ferner unsubstituiertes oder ein- oder mehrfach durch Fluor substituiertes 4,4'-Biphenylyl.

Z¹ und Z² sind bevorzugt Einfachbindungen, in zweiter Linie bevorzugt -0-C0-, -C0-0-, -C≡C- oder -CH₂CH₂-Gruppen.

Besonders bevorzugt für Z¹ ist -CO-O, -O-CO-, -C≡C- oder -CH₂CH₂-, insbesondere die -CH₂CH₂- und die -C≡C-Gruppe.

X bedeutet in den Verbindungen der vor- und nachstehenden Formeln Halogen oder CN, vorzugsweise Halogen. Halogen ist vorzugsweise Brom oder Chlor, insbesondere bevorzugt Chlor.

Besonders bevorzugt sind Verbindungen der Formel I, die eine Gruppe 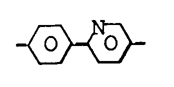
enthalten. Diese Verbindungen zeigen völlig überraschend wesentlich höhere Werte für die Spontanpolarisation als ähnliche Phenylpyrimidin- oder Biphenyl-Verbindungen.

Der Rest R¹-Q¹ ist ein mindestens einfach verzweigter und optisch aktiver organischer Rest mit einem asymmetrischen Kohlenstoffatom. Vorzugsweise ist das asymmetrische Kohlenstoffatom mit zwei unterschiedlich substituierten C-Atomen, einem H-Atom und einem Substituenten ausgewählt aus der Gruppe Halogen (insbesondere F, Cl oder Br) und CN verknüpft.
- X': ist vorzugsweise -CO-O-, -O-CO-, -CH=CH-COO- (trans) oder eine Einfachbindung. Besonders bevorzugt sind -CO-O-/-O-CO- oder eine Einfachbindung.
- Q': ist vorzugsweise -CH₂-, -CH₂CH₂-, -CH₂CH₂CH₂- oder eine Einfachbindung, insbesondere bevorzugt eine Einfachbindung.
- Y': ist Halogen oder -CN, vorzugsweise -CN oder Cl, insbesondere bevorzugt CN.
- R⁵: ist vorzugsweise geradkettiges oder verzweigtes Alkyl mit 1 bis 10, insbesondere mit 1 bis 7, C-Atomen.

Unter den Verbindungen der Formel I sowie Ia bis Iq sind diejenigen bevorzugt, in denen mindestens einer der darin enthaltenen Reste eine der angegebenen bevorzugten Bedeutungen hat.

Besonders bevorzugt sind Verbindungen der Formel I, worin R² eine geradkettige Alkyl- oder Perfluoralkyl-Gruppe mit jeweils 1-12, vorzugsweise 5-12, C-Atomen ist worin auch eine oder mehrere CH₂ - bzw. CF₂-Gruppen durch eine Gruppierung ausgewählt aus der Gruppe -O-, -S-, -CO-, -OCO-, -O-COO-, -CO-O- und -CH=CH- oder auch durch eine Kombination von zwei geeigneten Gruppierungen ersetzt sein können, wobei zwei Heteroatome nicht direkt miteinander verknüpft sind. R² bedeutet entsprechend vorzugsweise Alkyl, -O-Alkyl, -S-Alkyl, -O-CO-Alkyl oder -COO-Alkyl, worin Alkyl eine geradkettige Alkyl-, Alkenyl-, Perfluoralkyl- oder Polyfluoralkylgruppe mit jeweils 1-12 C-Atomen, vorzugsweise geradkettiges Alkyl, ist.

In den Verbindungen der Formel I sowie in den vor- und nachstehenden Teilformeln ist -A⁴-Z¹-A²-(Z²-A³)ₙ- vorzugsweise eine Gruppe der folgenden Formeln 1 bis 16 oder deren Spiegelbild: 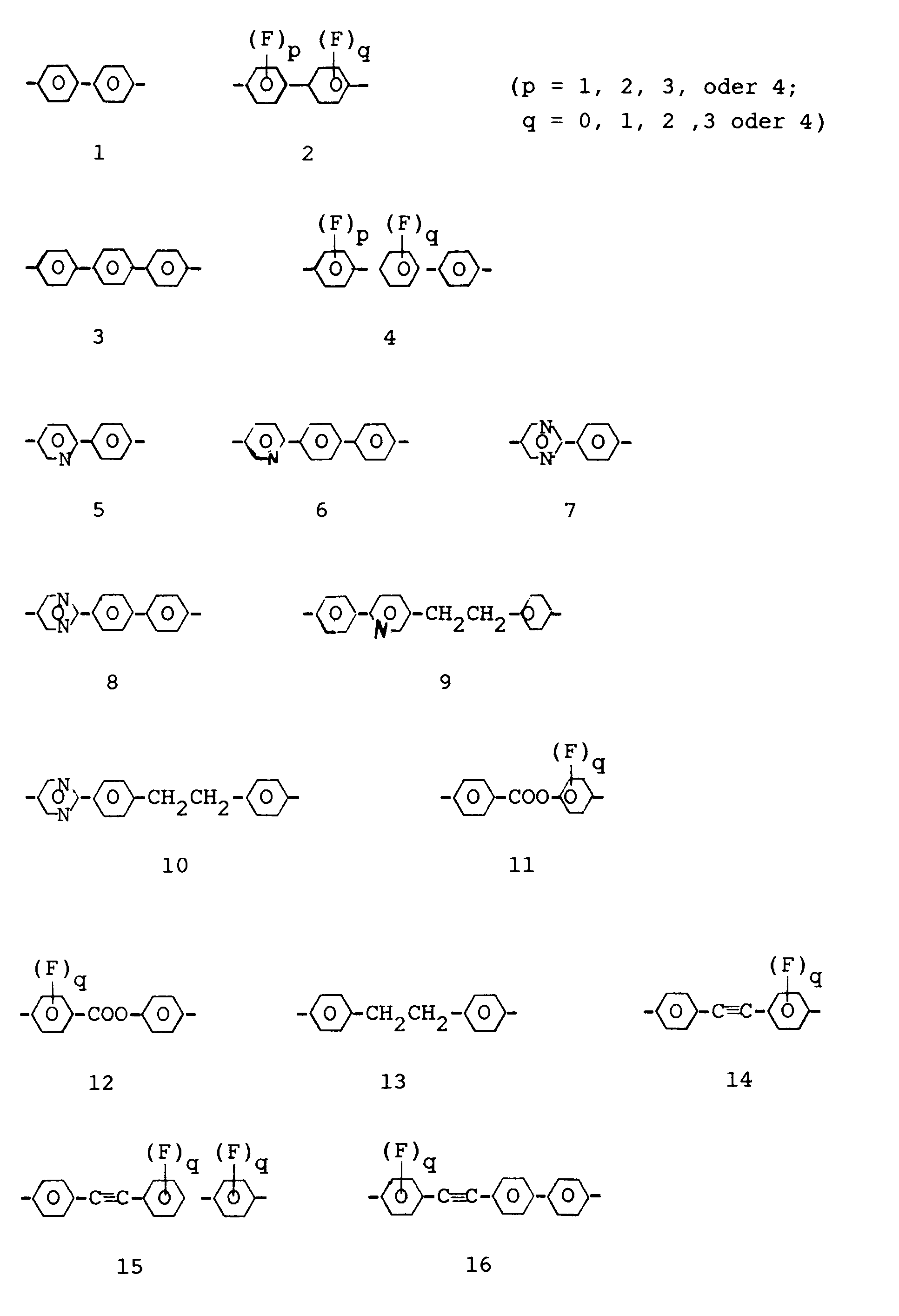
Gruppen der Formeln 1, 5, 7, 9, 10, 11, 12, 13 und 14, insbesondere diejenigen der Formeln 5 und 7, sind besonders bevorzugt.

Diejenigen der vorstehend genannten Formeln, die eine oder mehrere Gruppen Dio, Dit, Pip und/oder Pyr enthalten, umschließen jeweils die beiden möglichen 2,5-(Dio, Dit, Pyr) bzw. 1,4-Stellungsisomeren (Pip).

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise -CH=CH-gruppen in Betracht, ferner z.B. freie oder veresterte Hydroxygruppen, aromatisch gebundene Halogenatome oder Carbonylgruppen. Bevorzugte Ausgangsstoffe für die Reduktion entsprechen der Formel I, können aber an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit NaBH₄ oder Tributylzinnhydrid in Methanol hydriert werden; so entstehen z.B. aus 1-Cyancyclohexenderivaten die entsprechenden Cyclohexanderivate.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedinungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Dioxanderivate bzw. Dithianderivate der Formel I werden zweckmäßig durch Reaktion eines entsprechenden Aldehyds (oder eines seiner reaktionsfähigen Derivate) mit einem entsprechenden 1,3-Diol bzw. einem entsprechenden 1,3-Dithiol (oder einem ihrer reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, alle können ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Zur Herstellung von Nitrilen der Formel I können entsprechende Säureamide, z.B. solche in denen an Stelle des Restes X eine CONH₂-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCl₂, PCl₃, PCl₅, POCl₃, SO₂Cl₂, COCl₂, ferner P₂0₅, P₂S₅, AlCl₃ (z.B. als Doppelverbindungen mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol, oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Nitrilen der Formel I können auch entsprechende Chlor- oder Bromverbindungen der Formel I mit einem Cyanid umgesetzt werden, zweckmäßig mit einem Metallcyanid wie NaCN, KCN oder Cu₂(CN)₂, z.B. in Gegenwart von Pyridin in einem inerten Lösungsmittel wie DMF oder N-Methylpyrrolidon bei Temperaturen zwischen 20° und 200°.

Die optisch aktiven Verbindungen der Formel I erhält man durch den Einsatz entsprechender optisch aktiver Ausgangsmaterialien und/oder durch Trennung der optischen Antipoden mittels Chromatographie nach bekannten Methoden.

Die erfindungsgemäßen Phasen enthalten mindestens eine, vorzugsweise mindestens zwei Verbindungen der Formel I. Besonders bevorzugt sind erfindungsgemäße chiral getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der chiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vp: 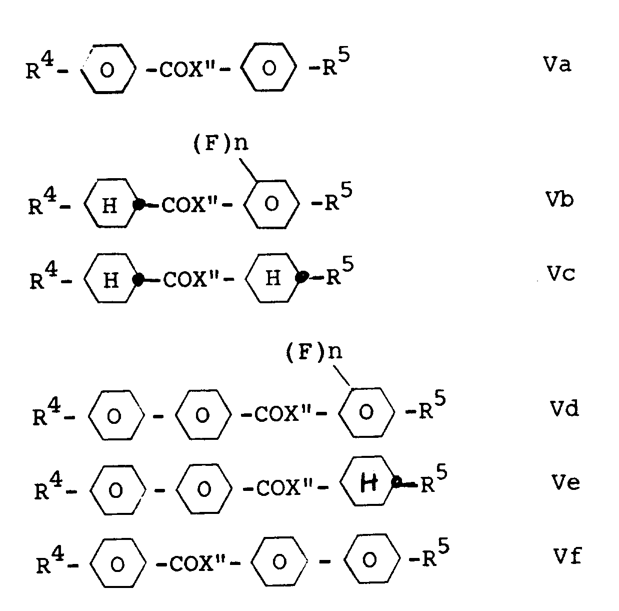 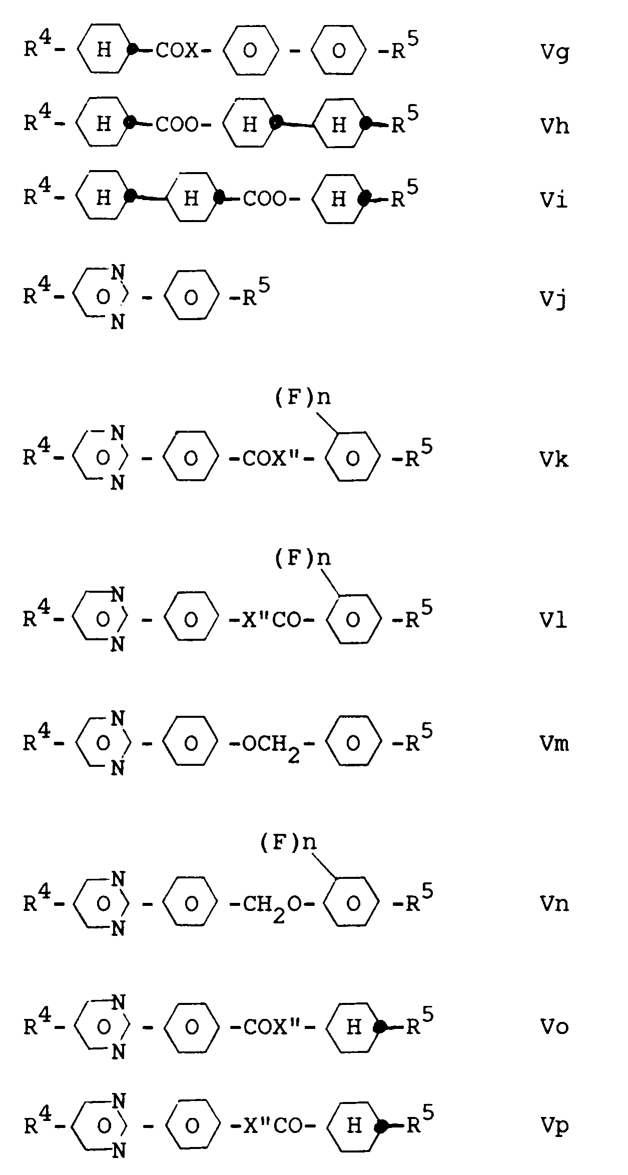
R⁴ und R⁵ sind jeweils vorzugsweise geradkettiges Alkyl, Alkoxy, Alkanoyloxy oder Alkoxycarbonyl mit jeweils 3 bis 12 C-Atomen. X'' ist O oder S, vorzugsweise O. n ist 0 oder 1.

Besonders bevorzugt sind die Verbindungen der Teilformeln Va, Vb, Vd und Vf, worin R⁴ und R⁵ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. R⁴ und R⁵ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel 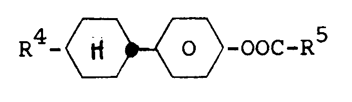
worin R⁴ und R⁵ die für Vc, Vh und Vi angegebene bevorzugte Bedeutung haben.

Als weitere Komponenten mit negativer dielektrischer Anisotropie eignen sich weiterhin Verbindungen enthaltend das Strukturelement M, N oder O. 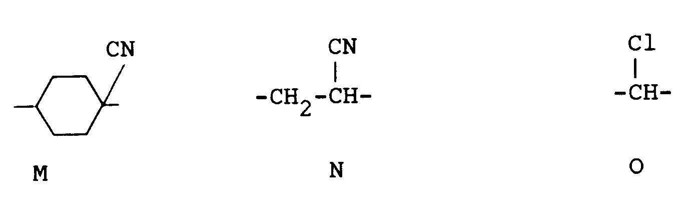
Bevorzugte Verbindungen dieser Art entsprechen den Formeln VIb und VIc:

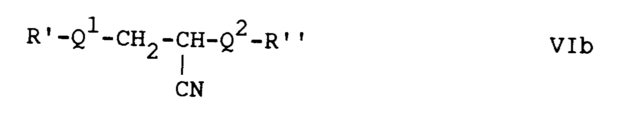

R'-Q³-Q⁴-R''' VIc

R' und R'' bedeuten jeweils vorzugsweise geradkettige Alkyl- oder Alkoxy-Gruppen mit jeweils 2 bis 10 C-Atomen. Q¹ und Q² bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen Q¹ und Q² auch eine Einfachbindung.

Q³ und Q⁴ bedeuten jeweils 1,4-Phenylen, 4,4'-Biphenylyl oder trans-1,4-Cyclohexylen. Eine der Gruppen Q³ und Q⁴ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur 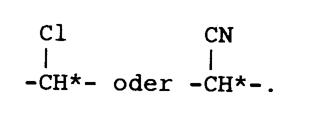
Besonders bevorzugte Verbindungen der Formel VIc sind diejenigen der Formel VIc': 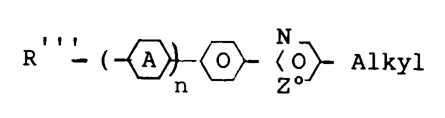
worin A 1,4-Phenylen oder trans-1,4-Cyclohexylen, Z° CH oder N und n 0 oder 1 bedeutet.

Die Verbindungen der Formel I eignen sich auch als Komponenten nematischer flüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden Vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I' charakterisieren,

R'-L-G-E-R'' I'

worin L und E je ein carbo- oder heterocyclisches Ring-system aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,
- G: -CH=CH- -N(0)=N-
-CH=CY- -CH=N(0)-
-C≡C- -CH₂-CH₂-
-C0-0- -CH₂-0-
-C0-S- -CH₂-S-
-CH=N- -C00-Phe-C00-
oder eine C-C-Einfachbindung,
Y Halogen, vorzugsweise Chlor, oder -CN, und
R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, N0₂, CF₃, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249 - 258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

## Patentansprüche

1. Optisch aktive Verbindungen der Formel I
R¹-Q¹-C*HX-Q²-A⁴-Z¹-A²-(Z²-A³)ₙ-R² I
worin
R² eine geradkettige oder verzweigte Alkyl- oder Perfluoralkyl-Gruppe mit jeweils 1-12 C-Atomen, worin auch eine oder mehrere CH₂- bzw. CF₂-Gruppen durch eine Gruppierung ausgewählt aus der Gruppe -O-, -S-, -CO-, -O-CO-, -S-CO-, -O-COO-, -CO-O-, -CO-S-, -CH=CH-, -CHHalogen- und -CHCN- oder auch durch eine Kombination von zwei geeigneten Gruppierungen ersetzt sein können, wobei zwei Heteroatome nicht direkt miteinander verknüpft sind, F, Cl, Br, CN oder auch H,
A², A³ und A⁴ jeweils unsubstituiertes oder durch ein oder zwei F- und/oder Cl-Atome und/oder CH₃-Gruppen und/oder CN-Gruppen substituiertes 1,4-Phenylen, worin auch eine oder zwei CH-Gruppen durch N ersetzt sein können, 1,4-Cyclohexylen, worin auch ein oder zwei nicht benachbarte CH₂-Gruppen durch O-Atome und/oder S-Atome ersetzt sein können, Piperidin-1,4-diyl, 1,4-Bicyclo(2,2,2)-octylen-, Naphthalin-2,6-diyl-, Decahydronaphthalin-2,6-diyl- oder 1,2,3,4-Tetrahydronaphthalin-2,6-diyl-Gruppen,
Z¹ und Z² jeweils -CO-O-, -CO-S-, -O-CO-, -S-CO-, -CH₂CH₂-, -OCH₂-, -CH₂O-, -C≡C- oder eine Einfachbindung,
X Halogen oder CN
n 0 oder 1,
Q² -(CH₂)ₚ-, -(CH₂)ₚ-CO-O, -(CH₂)ₚ-O-CO- oder -(CH₂)ₚ-O-, wobei p 1 bis 3 bedeutet, oder eine Einfachbindung
und
R¹-Q¹- 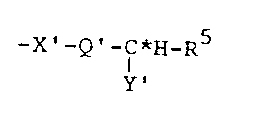 bedeutet,
worin
X' -CO-O-, -O-CO-, -O-CO-O-, -CO-, -O-, -S-, -CH=CH-, -CH=CH-COO- oder eine Einfachbindung,
Q' Alkylen mit 1 bis 5 C-Atomen, worin auch eine nicht mit X' verknüpfte CH₂-Gruppe durch -O-, -CO-, -O-CO-, -CO-O- oder -CH=CH- ersetzt sein kann, oder eine Einfachbindung,
Y' CN oder Halogen und
R⁵ eine Alkylgruppe mit 1 bis 15 C-Atomen, worin auch eine oder zwei nicht benachbarte CH₂-Gruppen durch -O-, -CO-, -O-CO-, -CO-O- und/oder -CH=CH- ersetzt sein können,
bedeutet.

2. Chirale getiltete smektische flüssigkristalline Phase mit mindestens zwei flüssigkristallinen Komponenten, dadurch gekennzeichnet, daß sie mindestens eine optisch aktive Verbindung der Formel 1 nach Anspruch 1 enthält.

3. Verwendung der Verbindungen der Formel I nach Anspruch 1 als Komponenten flüssigkristalliner Phasen.

4. Elektrooptisches Anzeigeelement, dadurch gekennzeichnet, daß es als Dielektrikum eine Phase nach Anspruch 2 enthalt.

## Claims

1. Optically active compounds of the formula I
R¹-Q¹-C*HX-Q²-A⁴-Z¹-A²-(Z²-A³)ₙ-R² I
in which
R² is a straight-chain or branched alkyl or perfluoroalkyl group with in each case 1-12 C atoms, in which one or more CH₂ or CF₂ groups can also be replaced by a grouping selected from the group comprising -O-, -S-, -CO-, -O-CO-, -S-CO-, -O-COO-, -CO-O-, -CO-S-, -CH=CH-, -CHhalogen- and -CHCN- or by a combination of two suitable groupings, two hetero atoms not being linked directly to one another, F, Cl, Br, CN or also H,
A², A³ and A⁴ are each 1,4-phenylene which is unsubstituted or substituted by one or two F and/or Cl atoms and/or CH₃ groups and/or CN groups, in which one or two CH groups can also be replaced by N, or 1,4-cyclohexylene, in which one or two non-adjacent CH₂ grups can also be replaced by O atoms and/or S atoms, or piperidine-1,4-diyl, 1,4-bicyclo(2,2,2)-octylene, naphthalene-2,6-diyl, decahydronaphthalene-2,6-diyl or 1,2,3,4-tetrahydronaphthalene-2,6-diyl groups,
Z¹ and Z² are each -CO-O-, -CO-S-, -O-CO-, -S-CO-, -CH₂CH₂-, -OCH₂-, -CH₂O, -C≡C or a single bond,
X is halogen or CN,
n is 0 or 1,
Q² is -(CH₂)ₚ-, -(CH₂)ₚ-CO-O-, -(CH₂)ₚ-O-CO- or -(CH₂)ₚ-O-, p being 1 to 3, or a single bond
and
R¹-Q¹- is 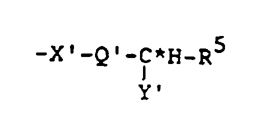
in which
X' is -CO-O-, -O-CO-, -O-CO-O-, -CO-, -O-, -S-, -CH=CH-, -CH=CH-COO- or a single bond,
Q' is alkylene with 1 to 5 C atoms, in which a CH₂ group not linked with X' can also be replaced by -O-, -CO-, -O-CO-, -CO-O- or -CH=CH-, or is a single bond,
Y' is CN or halogen and
R⁵ is an alkyl group with 1 to 15 C atoms, in which one or two non-adjacent CH₂ groups can also be replaced by -O-, -CO-, -O-CO-, -CO-O- and/or -CH=CH-.

2. Chiral tilted smectic liquid crystal phase with at least two liquid crystal components, characterized in that it contains at least one optically active compound of the formula 1 according to Claim 1.

3. Use of the compounds of the formula I according to Claim 1 as components of liquid crystal phases.

4. Electrooptical display element, characterized in that it contains a phase according to Claim 2 as the dielectric.

## Revendications

1. Composés possédant l'activité optique et répondant à la formule I
R¹-Q¹-C*HX-Q²-A⁴-Z¹-A²-(Z²-A³)ₙ-R²
dans laquelle
R² représente un groupe alkyle ou perfluoralkyle à chaîne droite ou ramifiée contenant chacun 1 à 12 atomes de carbone, dans lesquels également un ou plusieurs groupes -CH₂- ou -CF₂- peuvent être remplacés par un groupement choisi parmi -0-, -S-, -C0-, - 0-C0-, -S-C0-, -0-C00-, -C0-0-, -CO-S-, -CH=CH-, -CH halogène - et -CHCN- ou encore par une combinaison de deux groupements appropriés, sous réserve que deux hétéroatomes ne peuvent pas être reliés directement entre eux,
F, Cl, Br, CN ou encore H,
A², A³ et A⁴, représentent chacun un groupe 1,4-phénylène non substitué ou substitué par 1 ou 2 atomes de F et/ou de Cl et/ou groupes CH₃ et/ou groupes CN, et dans lequel également 1 ou 2 groupes CH peuvent être remplacés par N, un groupe 1,4-cyclohexylène dans lequel également un ou deux groupes CH₂ non voisins peuvent être remplacés par des atomes d'oxygène et / ou de soufre, des groupes pipéridine-1,4-diyle, 1,4-bi-cyclo (2,2,2)-octylène, naphtalène-2,6-diyle, décahydronaphtalène-2,6-diyle ou 1,2,3,4-tétrahydronaphtalène-2,6-diyle,
Z¹ et Z² représentent chacun -C0-0-, -CO-S-, -O-CO-, -S-CO-, -CH₂CH₂-, -OCH₂-, -CH₂O-, -C=C ou une liaison simple,
X représente un halogène ou CN
n est égal à 0 ou 1,
Q² représente -(CH₂)ₚ-, -(CH₂)ₚ-CO-O-, -(CH₂)ₚ-O-CO- ou - (CH₂)ₚ-O-, où p est un nombre compris entre 1 et 3 ou une liaison simple,
et
R¹-Q¹- représente 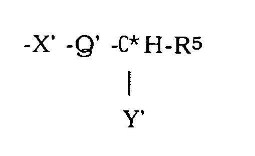 où X' représente -CO-O-, -O-CO-, -O-CO-O-, -CO-, -O-, -S-, - CH=CH, -CH=CH- COO- ou une liaison simple,
Q' représente un groupe alkylène en C 1-C 5 dans lequel également un groupe CH₂ non relié à X' peut être remplacé par -0-, - C0-, -0-C0- ou -CH=CH-, ou une liaison simple,
Y' représente un groupe CN ou un halogène, et
R⁵ représente un groupe alkyle en C 1-C 15 dans lequel également 1 ou 2 groupes CH₂ non voisins peuvent être remplacés par -0-, -C0-,
-0-C0-, -C0-0- et/ou -CH=CH-.

2. Phase chirale inclinée à cristaux liquides smectiques à au moins deux composants à cristaux liquides, caractérisée en ce qu'elle contient au moins un composé possédant l'activité optique et répondant à la formule I selon revendication 1.

3. Utilisation des composés de formule I de la revendication 1 en tant que composants de phases à cristaux liquides.

4. Elément d'affichage électro-optique, caractérisé en ce qu'il contient en tant que diélectrique une phase selon la revendication 2.
